(19) Europäisches Patentamt
European Patent Office
Office européen des brevets

(11) **EP 2 374 835 B1**

(12) **EUROPEAN PATENT SPECIFICATION**

(45) Date of publication and mention
of the grant of the patent:
**25.12.2013 Bulletin 2013/52**

(51) Int Cl.:
*C08J 3/12* (2006.01)     *C08J 3/14* (2006.01)
*C08L 77/06* (2006.01)     *A61Q 1/00* (2006.01)
*A61Q 3/00* (2006.01)     *A61K 8/02* (2006.01)
*A61K 8/88* (2006.01)     *A61Q 19/00* (2006.01)
*B29C 67/00* (2006.01)

(21) Application number: **11160957.4**

(22) Date of filing: **04.04.2011**

(54) **Polyamide 1010 powder and its use in personal care products**

Polyamid-1010-Pulver und dessen Verwendung in Körperpflegeprodukten

Poudre de polyamide 1010 et son utilisation dans des produits de soins personnels

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **07.04.2010 EP 10159183
09.02.2011 EP 11153777**

(43) Date of publication of application:
**12.10.2011 Bulletin 2011/41**

(73) Proprietor: **Evonik Degussa GmbH
45128 Essen (DE)**

(72) Inventors:
• **Jha, Brajesh Kumar
23112 Midlothian, VA (US)**
• **Meyer, Juergen
45134, Essen (DE)**
• **Gruening, Burghard
45134, Essen (DE)**
• **Baumann, Franz-Erich
48249, Dülmen (DE)**
• **Christoph, Wolfgang
45768, Marl (DE)**
• **Stemmer, Heike
45721, Haltern am See (DE)**
• **Warnke, Kristiane
45665, Recklinghausen (DE)**

(56) References cited:
**WO-A2-2009/101320     US-A1- 2008 116 616
US-A1- 2008 249 237**

• **"Polyamide-12 Powders for Beauty Care",
Degussa , 1 May 2003 (2003-05-01), XP002588878,
Retrieved from the Internet: URL:http:
//www.quetzalquimica.com/images/D S_
TEGOLON_e10-08-2007.pdf [retrieved on
2010-06-23]**

**Description**

The present invention is directed to special polyamide 1010 powders, particularly for use in personal care and cosmetic products, as well as to a method for producing such powder, and their use in personal care products and applications.

The use of polyamide powder in personal care products and applications is well known. Evonik Goldschmidt GmbH for example provides polyamide 12 powder for use in powder preparations, foundation, nail polishes and aerosols under the trade name TEGOLON® 12-10 and TEGOLON® 12-20 (TEGOLON® is a registered trademark of Evonik Goldschmidt GmbH). These powders have a particle size ($d_{50}$ value) of 6 and 10 $\mu$m respectively.

US 2003/0114636 (Degussa AG) describes the use of pH-regulated polyamide powders for cosmetic applications. The polyamide types described in this application are: polyamide 11 and polyamide 12. Polyamide (PA) of the AA.BB-type were not mentioned. The average particle size of the particles is of from 1 to 400 $\mu$m. The PA powder has a pH value of from 4 to 7.

EP 0 863 174 (equal to US 5,932,687) describes polyamide powders of narrow particle size distribution and low porosity and a precipitation process for producing the same. The polymer powders show a particle size below 100 $\mu$m, a BET surface below 10 $m^2/g$ and an apparent density of more than 400 g/l. In comparative example 5 the precipitation of polyamide 10.10 is described. At a precipitation temperature of 120 °C particles are obtained with an apparent density of 417 g/l and 99.7 % by weight of the particles have a particle size below 160 $\mu$m. In example 28 a two step precipitation process for producing polyamide 10.10 particles is performed. The precipitation temperature is again 120°C. The particles obtained have an apparent density of 440 g/l and 99.7 % by weight of the particles have a particle size below 160 $\mu$m. The polyamide particles obtained by this process are especially useful in metal coating processes.

EP 1 726 610 describes fine spherical thermoplastic resin particles that are useful as cosmetic materials. As thermoplastic resin particles are disclosed among others polyamide resins (PA resins). Explicitly mentioned are PA 4.6, PA 6.6, PA 6.12, PA 9.12, PA 12.12, PA 10.12, and PA 10.10 as polyamides of the AA.BB-type. The thermoplastic resin particles preferably have an average particle diameter of from 0.01 to 100 $\mu$m with a variation of the particle diameter of less than 30 %.

EP 1 834 979 describes resin particles for use as components in cosmetics. As resin particles are disclosed among others polyamide resins (PA resins). Explicitly mentioned are PA 4.6, PA 6.6, PA 6.12, PA 9.12, PA 12.12, PA 10.12, and PA 10.10 as polyamides of the AA.BB-type. The thermoplastic resin particles preferably have an average particle diameter of from 0.01 to 300 $\mu$m. The particles are produced by drying filter cake obtained by filtering a mixture of resin particles, a liquid material having a boiling point of 100°C or higher and water.

US 2008/0249237 describes a process for producing ultrafine powders based on polyamides. The powders are prepared by contacting polyamides having a relative solution viscosity of from 1.5 to 2.0 with an alcoholic medium in the presence of inorganic particles having a mean size ($d_{50}$) of from 0.001 to 0.8 $\mu$m. The PA powders obtained by this process comprise a BET surface of from 5 to 100 $m^2/g$, a mean size ($d_{50}$) of less than 70 $\mu$m, and an apparent density of from 250 to 1000 g/l. In example 8 a PA 10.10 powder is produced having a BET surface of 15.7 $m^2/g$, a mean size ($d_{50}$) of 21 $\mu$m, and an apparent density of 381 g/l. The use of such powder(s) for cosmetic applications is not described.

A problem of personal care products that contain oil or oily ingredients is the oily feeling on the skin after such products are applied to skin. Another problem with the current personal care products is the lack of versatility to deliver multiple application benefits, such as efficiency in sebum absorption apart from providing a feeling of greater slipperiness and smoothness and improved spreading on the skin.

The problem to be solved by the present invention was to provide personal care products, especially lotions, creams etc. that leave a lesser oily feeling after application on skin than the personal care products known in the art.

Surprisingly we found that if products that comprise the PA 10.10 powder as claimed in claim 1 are applied to skin the users do not observe an oily feeling of the skin or observe at least a lesser oily feeling.

One object of the present invention are therefore particles based on polyamide 10.10, characterized in that the particles have a mean particle size $d_{50}$ of from 1 to 50 $\mu$m, preferably 5 to 30 $\mu$m, an apparent density of from 120 to 300 g/l, preferably from 150 to 250 g/l. In a preferred embodiment the $NH_2$/COOH end group ratio ranges from 50 : 50 to 95 : 5.

Another object of the invention are formulations, especially cosmetic, dermatologic or pharmaceutical formulations comprising 0.1 to 20 % by weight of the particles according to the invention and the use / a method of use of particles according to the invention for producing cosmetic, dermatologic or pharmaceutical formulation, preferably selected from powder composition, foundation, nail polish, aerosol, lipstick, eye shadow, masking stick, rouge, skin cream face cream, hair care, sun care, cleansing, AP/Deo, etc.

A further object of the present invention is a process for preparing the particles of the invention by dissolving polyamide 10.10, having a relative solution viscosity $\eta_{rel}$ in the range from 1.4 to 2.0, measured in 0.5% m-cresol solution at 25°C, in an alcoholic medium, preferably in an aliphatic $C_1$- to $C_3$-alcohol, preferably under pressure, lowering the temperature in a first stage until nucleation takes place without precipitation, lowering the temperature further in a second stage until supersaturation results, precipitating said polyamide powder and drying the resulting suspension, wherein said polyamide 10.10 is dissolved at from 130 to 165 °C and precipitation is carried out isothermally at a precipitation temperature of from 100 to 130 °C preceded by a nucleation stage at from 2 to 20 °C above said precipitation temperature, wherein said temperature during precipitation is held constant for from 10 minutes to 2 hours, preferably for from 20 to 45 minutes, and wherein said temperature during nucleation is held constant for from 30 to 180 minutes, preferably of from 90 to 150 minutes. Preferably during nucleation the temperature is held constant in the range of from 122 to 128°C for from 30 to 45 minutes. Preferably during precipitation the temperature is held constant for 90 to 120 minutes at a range of from 117 to 121°C. It is preferred to slow the precipitation at higher temperature to achieve particles with a high BET value.

The PA 10.10 based particles of the present invention have the advantage that if they are used to produce oil or oily compounds containing personal care products after application of these products to skin these products do not leave or at least only leave a lesser oily feeling than products comprising PA powders known in the art.

The PA 10.10 particles of the present invention can be made completely from natural resources, especially from castor oil. The PA 10.10 particles of the present invention can therefore be produced environment-friendly from a renewable source and its production is therefore almost $CO_2$ neutral.

With reference to established nylon 11 or nylon 12 powders the higher hydrophilicity of PA10.10 provides a better moisture retention on the skin and easy dispersion in water continuous formulations. At the same time and with reference to more polar PA612- or PA610- powders a better oil absorption is advantageous in oil or fat-based formulations.

The particles according to the invention, their use and a process for producing the same are described below by way of example without any intention of limiting the invention to these exemplary embodiments. Where ranges, general formulae or compound classes are given below, then these are intended to encompass not only the corresponding ranges or groups of compounds explicitly mentioned, but also all part ranges and part groups of compounds which can be obtained by removing individual values (ranges) or compounds. Where documents are cited within the context of the present description, then it is intended for their content, in its entirety, to form part of the disclosure of the present invention. Unless stated otherwise, all of the data in per cent (%) are per cent by mass. Unless stated otherwise, all of the average values which may be stated are number averages.

The particles based on polyamide 10.10 according to the invention are characterized by having a mean particle size $d_{50}$ of from 1 to 50 $\mu$m, preferably of from 5 to 30 $\mu$m, an apparent density of from 180 to 300 g/l, preferred 200 to 230 g/l, and a $NH_2$/COOH end group ratio of from 50 : 50 to 95 : 5, preferably 75 : 25 to 90 : 10.

Powders with an excess of COOH terminal groups may be equipped with a buffer system according to DE 101 61 038 A1.

The $NH_2$/COOH end group ratio is determined by known methods by alkalimetric titration with KOH in hot benzylic alcohol at 180°C and by acidimetric titration with $HClO_4$ at ambient temperature in m-Cresol.

The mean particle size $d_{50}$ is preferably determined by light scattering in a laser beam with a Malvern Mastersizer 2000. The determination is done using the dry measurement. Each time 20 to 40 g powder are fed using a Scirocco dry powder feeder. The particle flow is controlled operating the vibrating tray with a feed-rate of 70 %. The dispersive air pressure is adjusted to be 3 bar. Each measurement is accompanied by a measurement of the background (10 seconds / 10,000 single measurements). The measurement time of the sample is 5 seconds (5,000 single measurements). The refraction index as well as the blue light value are fixed to be 1.52. The evaluation is done using the Mie-theory.

The apparent density is determined according to DIN 53644.

It might be advantageous if the particles according to the invention are characterized in that the pH of the polyamide 10.10 based particles is of from 2 to 7, preferably of from 4 to 7 and most preferably of from 4.5 to 6.5. The pH of the particles is determined in suspension of 1 g of the particles of the invention in 100 ml of distilled water. After stirring for 24 h the pH is determined using a calibrated pH electrode.

To make sure that the particles according to the invention have a defined pH it might be helpful, necessary or advantageous when the particles comprise a buffer system or at least the remains of a buffer system. Preferably the buffer system comprises an organic acid, preferably a natural organic acid, or a mineral acid, preferably a natural mineral acid, and a corresponding salt thereof.

Preferred particles according to the invention are characterized in having a BET surface of from 1 to 60, preferably 1.5 to 20 m²/g, more preferably of from 3 to 10 m²/g. Particles according to the invention having such a low BET surface value are advantageously used in formulations with low oil phase content to provide good texture and pleasant sensory skin feel.

Other preferred particles according to the invention are characterized in having a BET surface of more than 20 m²/g, preferably from 25 to 200 m²/g, more preferably from 40 to 72 m²/g. Particles according to the invention having such a high BET surface value are especially able to absorb a high amount of oil or oily ingredients. Therefore products comprising the high BET surface particles do not exhibit an oily appearance after application to skin.

The BET surface area is determined in compliance with DIN ISO 9277:2003-05 using the discontinuous volumetric process according to chapter 6.3.1 of DIN ISO 9277:2003-05 using the gas adsorption apparatus TriStar 3000 of Micromeritics (Software Win 3000, V6.03) with continuous gas supply by absorption of nitrogen according to Brunauer-Emmett-Teller. The nitrogen used has a purity of 99.996 % by volume. The determination is done at a measurement temperature 77 K (liquid nitrogen) using several (seven) single point measurements at a relative pressure $p/p_0$ between about 0.05 and 0.20. The calibration of the dead volume is done using helium with a purity of 99.996 % by volume. The samples were degassed for 1 hour at 25 °C and for 16 hours at 80 °C afterwards under vacuum. The specific surface area given is based on the degassed sample. The interpretation was done according to DIN ISO 9277:2003-05, Chapter 7.2 by multipoint determination.

The particles according to the present invention, especially the particles having a BET surface of more than 20 $m^2$/g, preferably have an ability to absorb more than 90 ml/g, more preferably more than 100 ml/g of oil tested with common cosmetic oils according to standard methods.

This method determines the oil absorption capability of particles and is applicable to all microporous particles with an oil absorption capability. In this method, 5 grams of the particle which has been thoroughly mixed and air-dried is placed upon the watch glass. The oil (a caprylic/capric triglyceride sold under the trade name TEGOSOFT® CT by Evonik Goldschmidt GmbH) is accurately weighed in the dropping bottle apparatus. The oil is added drop by drop to the sample. After the addition of each drop, the oil is thoroughly incorporated with the powder by rubbing with a sharp-edged steel spatula. The test is complete when exactly enough oil has been incorporated with the powder to produce very stiff putty-like paste which does not break and separate. The dropping bottle is accurately weighed. The oil absorption capability of particle is calculated by the following equation:

$$\texttt{Oil absorption (g oil/sample) = (B - A)/W}$$

where

A = initial weight of the dropping bottle with oil,
B = final weight of the dropping bottle with oil and
W = weight of the sample in grams.

Especially preferred particles according to the invention are characterized in having a BET surface of from 3 to 10 $m^2$/g or from more than 10 to 20 $m^2$/g and a mean particle size $d_{50}$ of from 5 to 30 $\mu$m.

The mean molecular weight of the polyamide 10.10 of the particles according to the invention can vary over a broad range. Preferably the mean number average molecular weight of the polyamide 10.10 is of from 5000 to 50000 g/mol, preferably 8000 to 20000 g/mol determined by terminal group titration. The mean weight average molecular weight is preferably between 10000 and 200000, more preferred between 15000 and 50000 determined by GPC.

It might be advantageous if the particles of the invention comprise at least one inorganic particular material. Preferred particles according to the invention are characterized in a content of inorganic particles of from 0.1 to 80 % by weight, preferably 1 to 60 % by weight and more preferably of from 25 to 50 by weight, based on the total weight of the particles. The inorganic particles can be present in the inside of the particles or be bound to the surface of the particles of the invention. The presence of inorganic particles can have a significant influence on the BET-Surface of the particles according to the invention. The content of inorganic particles might be determined by an ash/ignition residue determination according to DIN EN ISO 3451 Part 1 and Part 4.

The particles of the invention feature a unique combination of properties. In addition to the properties mentioned, they also possess a relatively narrow particle size distribution, which is evident from the examples. Owing to their outstanding properties, the powders are suitable for a whole series of applications. The particles of the invention can for example be used as a coating composition. It is possible to use the particles of the invention either as fluidized-bed sintering powders or as electrostatic powders. The particles of the invention are equally outstandingly suitable for the production of mouldings and components.

The particles of the invention are preferably used for producing (or in a method to produce) formulations, especially cosmetic, dermatologic or pharmaceutical formulation, preferably selected from powder composition, foundation, nail polish, aerosol, lipstick, eye shadow, masking stick, rouge, skin cream, face cream, hair care, sun care, cleansing, AP/Deo, etc.

The preparation of the formulation, especially the cosmetic, dermatologic or pharmaceutical formulation can be done as known in the art. Preferably the cosmetic product is produced by adding 0.1 to 20 % by weight, preferably 1 to 10 % by weight based on the total composition of the formulation.

The formulations of the present invention, especially the cosmetic, dermatologic or pharmaceutical formulations are characterized in that they comprise 0,1 to 20 % by weight, preferably 1 to 10 % by weight of particles according to the invention. Preferred cosmetic, dermatologic or pharmaceutical formulations are selected from powder compositions, foundations, nail polishes, aerosols, lipsticks, eye shadows, masking sticks, rouge, skin creams, face cream, hair care formulations, sun care formulations, cleansing formulations and AP/Deo formulations.

The formulations of the invention, especially the cosmetic, dermatological or pharmaceutical formulations of the invention can, for example, comprise at least one additional component selected from the group of

emollients,
emulsifiers and surfactants,
thickeners/viscosity regulators/stabilizers,
UV photoprotective filters,
UV photoprotective particulate materials,
antioxidants,
hydrotropes
polyols,
solids and fillers,
film formers,
pearlescent additives,
deodorant and antiperspirant active ingredients,
insect repellents,
self-tanning agents,
agents which influence the skin pigmentation,
preservatives,
conditioners,
perfumes,
dyes,
cosmetic active ingredients,
care additives,
cosmetic particles (e.g., elastomers, PMMA,
polyamide, wax, starch, etc.)
superfatting agents,
solvents.

Substances which can be used as exemplary representatives of the individual groups can be found in the German application DE 102008001788.4. This patent application is hereby incorporated by reference and thus forms part of the disclosure.

Emollients which can be used are all cosmetic oils, in particular mono- or diesters of linear and/or branched mono- and/or dicarboxylic acids having 2 to 44 carbon atoms with linear and/or branched saturated or unsaturated alcohols having 1 to 22 carbon atoms. The esterification products of aliphatic, difunctional alcohols having 2 to 36 carbon atoms with monofunctional aliphatic carboxylic acids having 1 to 22 carbon atoms can likewise be used. Also suitable are long-chain aryl acid esters, such as, for example, esters of benzoic acid, e.g. benzoic acid esters of linear or branched, saturated or unsaturated alcohols having 1 to 22 carbon atoms, or else isostearyl benzoate or octyldodecyl benzoate or for example $C_{12-15}$-alkyl benzoate, or esters of benzoic acid with linear or branched $C_6$-$C_{22}$-alcohols. Further monoesters suitable as emollients and oil components are, for example, the methyl esters and isopropyl esters of fatty acids having 12 to 22 carbon atoms, such as, for example, methyl laurate, methyl stearate, methyl oleate, methyl erucate, isopropyl palmitate, isopropyl myristate, isopropyl stearate, isopropyl oleate. Other suitable monoesters are, for example, n-butyl stearate, n-hexyl laurate, n-decyl oleate, isooctyl stearate, isononyl palmitate, isononyl isononanoate, 2-ethylhexyl palmitate, 2-ethylhexyl laurate, 2-hexyldecyl stearate, 2-octyldodecyl palmitate, oleyl oleate, oleyl erucate, erucyl oleate, and also esters which are obtainable from technical-grade aliphatic alcohol cuts and technical-grade, aliphatic carboxylic acid mixtures, e.g. esters of unsaturated fatty alcohols having 12 to 22 carbon atoms and saturated and unsaturated fatty acids having 12 to 22 carbon atoms, as they are accessible from animal and vegetable fats. Also suitable are naturally occurring monoester and/or wax ester mixtures as they are present, for example, in jojoba oil or in sperm oil. Suitable dicarboxylic acid esters are, for example, di-n-butyl adipate, di-n-butyl sebacate, di(2-ethylhexyl) adipate, di(2-hexyldecyl) succinate, diisotridecyl azelate. Suitable diol esters are, for example, ethylene glycol dioleate, ethylene glycol diisotridecanoate, propylene glycol di(2-ethylhexanoate), butanediol diisostearate, butanediol dicaprylate/caprate and neopentyl glycol dicaprylate. Further emollients which can be used are carbonates as for example dicaprylyl carbonate or diethylhexyl carbonate. Emollients and oil components which can likewise be used are relatively long-chain triglycerides, i.e. triple esters of glycerol with three acid molecules, of which at least one is relatively long-chain. Mention may be made here, by way of example, of fatty acid triglycerides; as such, it is possible to use, for example, natural, vegetable oils, e.g. olive oil, sunflower oil, soybean oil, peanut oil, rapeseed oil, almond oil, sesame oil, avocado oil, castor oil, cocoa butter, palm oil, but also the liquid fractions of coconut oil or of palm kernel oil, and also animal oils, such as, for example, shark liver oil, cod liver oil, whale oil, beef tallow and butter fat. Moreover waxes such as beeswax, carnauba palm wax, spermaceti, lanolin and claw oil, the liquid fractions of beef tallow and also synthetic triglycerides of caprylic/ capric acid mixtures, triglycerides of technical-grade oleic acid, triglycerides with isostearic acid, or from palmitic acid/ oleic acid mixtures may be used as emollients and oil components. Furthermore, hydrocarbons, in particular also liquid paraffins and isoparaffins, can be used. Examples of hydrocarbons which can be used are paraffin oil, isohexadecane, polydecene, vaseline, paraffinum perliquidum, squalane, ceresine. Furthermore, it is also possible to use linear or branched fatty alcohols such as oleyl alcohol or octyldodecanol, and also fatty alcohol ethers such as dicaprylyl ether.

Suitable silicone oils and silicone waxes are, for example, polydimethylsiloxanes, cyclomethylsiloxanes, and also aryl- or alkyl- or alkoxy-substituted polymethylsiloxanes or cyclomethylsiloxanes. Suitable further oil bodies are, for example, Guerbet alcohols based on fatty alcohols having 6 to 18, preferably 8 to 10, carbon atoms, esters of linear $C_6$-$C_{22}$-fatty acids with linear $C_6$-$C_{22}$-fatty alcohols, esters of branched $C_6$-$C_{13}$-carboxylic acids with linear $C_6$-$C_{22}$-fatty alcohols, esters of linear $C_6$-$C_{22}$-fatty acids with branched $C_8$-$C_{18}$-alcohols, in particular 2-ethylhexanol or isononanol, esters of branched $C_6$-$C_{13}$-carboxylic acids with branched alcohols, in particular 2-ethylhexanol or isononanol, esters of linear and/or branched fatty acids with polyhydric alcohols (such as, for example, propylene glycol, dimerdiol or trimertriol) and/or Guerbet alcohols, triglycerides based on $C_6$-$C_{10}$-fatty acids, liquid mono-/di-/triglyceride mixtures based on C6-C18-fatty acids, esters of $C_6$-$C_{22}$-fatty alcohols and/or Guerbet alcohols with aromatic carboxylic acids, in particular benzoic acid, vegetable oils, branched primary alcohols, substituted cyclohexanes, linear $C_6$-$C_{22}$-fatty alcohol carbonates, Guerbet carbonates, dialkyl ethers, ring-opening products of epoxidized fatty acid esters with polyols.

UV photoprotective filters which can be used are, for example, organic substances which are able to absorb ultraviolet rays and release the absorbed energy again in the form of longer-wave radiation, e.g. heat. UVB filters may be oil-soluble or water-soluble. Oil-soluble UVB photoprotective filters to be mentioned are, for example:

3-benzylidenecamphor and derivatives thereof, e.g. 3-(4-methylbenzylidene)camphor,
4-aminobenzoic acid derivatives, such as, for example, 2-ethylhexyl 4-(dimethylamino)benzoate and amyl 4-(dimethylamino)benzoate,
esters of cinnamic acid, such as, for example, 2-ethylhexyl 4-methoxycinnamate, isopentyl 4-methoxycinnamate, 2-ethylhexyl 2-cyano-3-phenylcinnamate (octocrylene),
esters of salicylic acid, such as, for example, 2-ethylhexyl salicylate, 4-isopropylbenzyl salicylate, homomenthyl salicylate,
derivatives of benzophenone, such as, for example, 2-hydroxy-4-methoxybenzophenone, 2-hydroxy-4-methoxy-4'-methylbenzophenone, 2,2'-dihydroxy-4-methoxybenzophenone,
esters of benzalmalonic acid, such as, for example, di-2-ethylhexyl 4-methoxybenzmalonate,
triazine derivatives, such as, for example, 2,4,6-trianilino-(p-carbo-2'-ethyl-1'-hexyloxy)-1,3,5-triazine and octyltriazone,
propane-1,3-diones, such as, for example, 1-(4-tert-butylphenyl)-3-(4'-methoxyphenyl)propane-1,3-dione.

Suitable water-soluble UVB photoprotective filters are: 2-phenylbenzimidazole-5-sulphonic acid and alkali metal, alkaline earth metal, ammonium, alkylammonium, alkanolammonium and glucammonium salts thereof, sulphonic acid derivatives of benzophenone, such as, for example, 2-hydroxy-4-methoxybenzophenone-5-sulphonic acid and its salts, sulphonic acid derivatives of 3-benzylidenecamphor, such as, for example, 4-(2-oxo-3-bornylidenemethyl)benzenesulphonic acid and 2-methyl-5-(2-oxo-3-bornylidene)sulphonic acid and salts thereof.

Suitable typical UVA photoprotective filters are, in particular, derivatives of benzoylmethane, such as, for example, 1-(4'-tert-butylphenyl)-3-(4'-methoxyphenyl)propane-1,3-dione or 1-phenyl-3-(4'-isopropylphenyl)propane-1,3-dione. The UV-A and UV-B filters can of course also be used in mixtures.

Besides the specified soluble substances, insoluble pigments are also suitable for this purpose, namely finely disperse metal oxides or salts, such as, for example, titanium dioxide, zinc oxide, iron oxide, aluminium oxide, cerium oxide, zirconium oxide, silicates (talc), barium sulphate and zinc stearate. The particles here should have an average diameter of less than 100 nm, e.g. between 5 and 50 nm and in particular between 15 and 30 nm. They may have a spherical shape, although it is also possible to use those particles which have an ellipsoidal shape or a shape which deviates in some other way from the spherical form. A relatively new class of photoprotective filters are micronized organic pigments, such as, for example, 2,2'-methylenebis{6-(2H-benzotriazol-2-yl)-4-(1,1,3,3-tetramethylbutyl)phenol} with a particle size of < 200 nm, which is obtainable, for example, as 50% strength aqueous dispersion.

Further suitable UV photoprotective filters can be found in the overview by P. Finkel in SÖFW-Journal 122, 543 (1996).

Besides the two aforementioned groups of primary UV photoprotective filters, it is also possible to use secondary photoprotective agents of the antioxidant type which interrupt the photochemical reaction chain which is triggered when UV radiation penetrates into the skin. Antioxidants which can be used are, for example, superoxide dismutase, tocopherols (vitamin E), dibutylhydroxytoluene and ascorbic acid (Vitamin C).

In one preferred embodiment, the cosmetic, dermatological or pharmaceutical formulations according to the invention comprise as additional component particles or pigments, preferably those selected from the group titanium dioxide, zinc oxide, iron oxide, aluminium oxide, zirconium oxide, silicates (talc), and zinc stearate, nylon-12, boron nitride, polyacrylate or polymethyl acrylate particles or silicone elastomers.

In a likewise preferred embodiment, the cosmetic, dermatological or pharmaceutical formulations according to the invention comprise as additional component cosmetic or biogenic active ingredients, preferably those selected from the group: phytosphingosine (and phytosphingosin derivatives), sphingosine (and sphingosine derivatives), sphingolipids, tocopherol, tocopherol acetate, tocopherol palmitate, ascorbic acid, polyphenols, deoxyribonucleic acid, coenzyme Q10, retinol, AHA acids, amino acids, hyaluronic acid, alpha-hydroxy acids, flavones, isoflavones, stilbenes, catechines, polyglutamic acid, creatine (and creatine derivatives), guanidine (and guanidine derivatives), pseudoceramides, essential oils and fatty acids, peptides, preferably peptides comprising from 2 to 10 amino acids, oligopeptides, protein hydrolysates, plant extracts, bisabolol, allantoin, panthenol, phytantriol, idebenone, liquorice extract, plant extracts, glycyrrhizidine and idebenone, scleroglucan, β-glucan, santalbic acid and vitamin complexes.

Examples of plant extracts are horsechestnut extract, camomile extract, rosemary extract, black and red currant extract, birch extract, rosehip extract, licorice extract, algae extract, green tea extract, aloe extract, ginger extract, ginseng extract, ginkgo extract, grapefruit extract, calendula extract, camphor, curcuma extract, thyme extract, mangosteen extract, amla extract, cystus extract, terminalia arjuna extract, oat extract, oregano extract, raspberry extract, strawberry extract, etc.

The biogenic active ingredients can also include the so-called barrier lipids, examples of which being ceramides, phytosphingosine and derivatives, sphingosine and derivatives, sphinganine and derivatives, pseudoceramides, phospholipids, lysophospholipids, cholesterol and derivatives, cholesteryl ester, free fatty acids, lanolin and derivatives, squalane, squalene and related substances.

Within the context of the invention, the biogenic active ingredients also include anti-acne, such as, for example, benzyl peroxide, phytosphingosine and derivatives, niacinamide hydroxybenzoate, nicotinaldehyde, retinol acid and derivatives, salicylic acid and derivatives, citronellic acid etc., and anti-cellulite, such as, for example, xanthine compounds such as caffeine, theophylline, theobromine and aminophylline, carnitine, carnosine, salicyloyl phytosphingosine, phytosphingosines, santalbic acid etc., as well as antidandruff agents such as, for example, salicylic acid and derivatives, zinc pyrithione, selenium sulphide, sulphur, cyclopiroxolamine, bifonazole, climbazole, octopirox and actirox etc., as well as astringents, such as, for example, alcohol, aluminium derivatives, gallic acid, pyridoxine salicylate, zinc salts, such as, for example, zinc sulphate, acetate, chloride, lactate, zirconium chlorohydrates etc. Bleaches such as kojic acid, arbutin, vitamin C and derivatives, hydroquinone, turmeric oil, creatinine, sphingolipids, oxyresveratrol, niacinamide, etc. may likewise be included in the biogenic active ingredients.

The dermatological or pharmaceutical formulations according to the invention may comprise alone or in combination with one or more of the actives mentioned above actives for antiperspirant or deodorant applications, as for example antiperspirants, esterase inhibitors, bactericidal or bacteriostatic agents, perspiration-absorbing substances and/or perfumes. Examples of those actives are given for example in US 2003053970.

The PA 10.10 particles of the invention may be used in cosmetic formulations combined with other cosmetic particle materials, such as PMMA, PS, PE, PP, Talcum, Silicone elastomers, silica, Mica and Boron nitride, etc. These particles can be soft or hard and can be nonporous to porous structures. Examples of typically silicone elastomeric particles or gels are Dow Corning 9040 Silicone Elastomers Blend and Dow Corning 9041 Silicone Elastomer Blend (Dow Corning) or KSG-15 or KSG-18 (Shin-Etsu).

The particles of the invention might be obtained by processes known in the art, providing that polyamide 10.10 is used in these processes. The particles of the present invention might especially be produced in a similar way as described in US 2003/0114636, US 2008/024937, DE 44 21 454 and/or EP 0 863 174.

The particles of the invention are preferably produced by the process of the invention. The process for preparing particles according to the invention is characterized the steps of dissolving polyamide 10.10, having a relative solution viscosity $\eta_{rel}$ in the range from 1.4 to 2.0, preferred 1.45 to 1.8 and more preferred from 1.5 to 1.6, measured in 0.5% m-cresol solution at 25°C, in an alcoholic medium, preferably in an aliphatic $C_1$- to $C_3$-alcohol, preferably under pressure, lowering the temperature in a first stage until nucleation takes place without precipitation, lowering the temperature further in a second stage until supersaturation results, precipitating said polyamide powder and drying the resulting suspension, wherein said polyamide 10.10 is dissolved at from 130 to 165 °C and precipitation is carried out isothermally at a precipitation temperature of from 100 to 130 °C preceded by a nucleation stage at from 2 to 20 °C above said precipitation temperature, wherein said temperature during nucleation is held constant for from 10 minutes to 2 hours, preferably 20 to 45 minutes, or wherein said temperature during nucleation is held constant for from 90 to 150 minutes. To obtain particles having a higher BET value it is preferred to slower precipitation at higher temperature.

In a preferred embodiment of the invention in the dissolving step 0.1 to 40 % by weight, preferably 12 to 25 % by weight, more preferably 14 to 17 % by weight, of inorganic particles are added to the alcoholic medium, based on the total weight of the sum of polyamide 10.10 and inorganic particles used.

The nature of the compounds usable as inorganic particles can vary over a wide range. Preferred inorganic particles are selected from the group consisting of $Al_2O_3$, $TiO_2$, $ZrO_2$, $SiO_2$, ZnO, $Bi_2O_3$, $CeO_2$, ITO (indium oxide doped with tin(IV) oxide), ATO (tin(IV) oxide doped with antimony oxide), IZO (indium oxide doped with zinc oxide), boron nitride, boron carbide, mixed oxides and spinels. Particular preference is given to the use of aluminium oxide ($Al_2O_3$) .

In this connection, the aluminium oxide may preferably be of pyrogenic origin. Pyrogenic means that corresponding aluminium oxide powder is obtained by reacting a suitable starting material in a flame. Pyrogenic processes include flame oxidation and flame hydrolysis. A particular process used for the industrial scale preparation of aluminium oxide is the flame hydrolysis of aluminium chloride in a hydrogen/oxygen flame. In general, the aluminium oxide particles prepared in this way are present in the form of aggregated primary particles, the primary particles being free of pores and bearing hydroxyl groups on their surface. In the reaction of aluminium chloride to give aluminium oxide, a by-product formed is hydrochloric acid which adheres to the aluminium oxide particles. Commonly, a majority of the hydrochloric acid is removed from the particles by a treatment with steam.

Aluminium oxide powders particularly suitable for the process of the invention include: AEROXIDE® Alu C, AEROXIDE® Alu 65, AEROXIDE® Alu 130, all Degussa AG, SpectrAl™ 100 Fumed Alumina, SpectrAl™ 51 Fumed Alumina, SpectrAl™ 81 Fumed Alumina, all Cabot Corp.

The inorganic materials used in the process of the invention preferably have a mean particle size $d_{50}$ of from 0.001 to 0.8 $\mu$m, preferably of from 0.005 to 0.5 $\mu$m and most preferably of from 0.01 to 0.3. The particle size as specified can be determined by known measurement methods by means of static or dynamic light scattering in a suspension of the particles. The values obtained via light scattering processes may be isolated particles or else agglomerates of primary particles in the suspension. What is important for the invention is that the particles actually present in the suspension, whether they be primary particles or agglomerates, have a $d_{50}$ value within the range specified. The particle size can be measured, for example, with a Zetasizer 3000 Hsa (Malvern Instruments, UK). When the particle size is above a $d_{50}$ value of 0.8 $\mu$m, the particle obtained by the process might under some circumstances be excessively large.

It might be advantageous to use inorganic particles having a specific surface area in the range from 5 to 200 $m^2$/g.

The inorganic particles are preferably added to the medium by adding a suspension comprising alcohol and the inorganic particles, wherein a suspension is used which preferably has a content of inorganic particles in the range from 10 to 60% by weight, preferably from 20 to 50% by weight, based on the total weight of the suspension.

The suspensions utilizable for the invention are generated with alcohol. This may be a pure alcohol, a mixture of a plurality of alcohols or else alcohols having a content of water or other substances which essentially do not disadvantageously influence the desired reprecipitation of the polyamides. The alcohol medium of the suspensions preferably has a content of less than 50% by weight of nonalcoholic substances (preferably water), more preferably less than 10% by weight and particularly appropriately less than 1% by weight of extraneous, nonalcoholic substances. Useful substances for the invention are generally all types of alcohols or mixtures thereof which permit reprecipitation of the polyamides under the desired conditions (pressure and temperature). In the individual case, it is possible for the person skilled in the art to adjust the system to specific requirements without any great complication. For the process of the invention, the alcoholic medium used for the reprecipitation of the polyamide and/or the suspension of the inorganic particles is preferably one or more alcohols which have a numerical ratio of oxygen atoms to carbon atoms in the range from 1:1 to 1:5.

Typical alcohols for preparing the suspension of the inorganic particles are those having a ratio of oxygen to carbon of 1:1, 1:2, 1:3, 1:4 and 1:5, preferably those having an oxygen to carbon ratio of 1:2 and 1:3, more preferably having an oxygen to carbon ratio of 1:2. The alcohol used to make the suspension is preferably the same alcohol as the alcohol present in the alcohol medium. Very particularly appropriately, ethanol is used in the preparation of a suspension of the inorganic particles, and in the reprecipitation of the polyamides.

To obtain a suspension, the particles are distributed finely in the alcoholic medium. This can be done by processes known in the art. Particular preference is given to processes which enable a high energy input. Such processes are described, for example, in DE 103 60 766 or DE 10 2005 032 427.4. In a preferred embodiment, the process of the invention is characterized in that a suspension is used which is obtainable by suspending the inorganic particles in the alcohol with introduction of an energy input of greater than 1000 kJ/$m^3$. This generally gives rise to very usable suspensions of the particles in the alcohol. The energy input addressed can be accomplished by known units. Suitable units may be: planetary kneaders, rotor-stator machines, stirred ball mills, roll mills and the like.

A particularly suitable procedure has been found to be one in which the suspension is first prepared with an energy input of less than 1000 kJ/$m^3$ to form a presuspension, the presuspension is divided into at least two substreams, these substreams are placed under a pressure of at least 500 bar in a high-energy mill, decompressed through a nozzle and allowed to meet one another in a gas- or liquid-filled reaction chamber, and the high-energy grinding is optionally repeated once or more than once.

The suspensions of inorganic particles in alcohol involved in the process according to the invention should be highly stable. In the context of the invention, particularly stable is understood to mean the stability of the suspension against sedimentation and reagglomeration within a period of one month, generally of at least six months.

To achieve particularly stable suspensions, it has also been found to be particularly advantageous when, in the distribution of the inorganic particles in the alcoholic medium, additives are present which can stabilize the suspension.

Such additives are, for example, phosphoric acid and its mono- or dibasic phosphates, phosphoric esters, phosphonic acids, organically modified phosphonic acid, sulphuric acid and derivatives thereof, nitric acid, generally organic mineral acids. In addition, it is also possible to use organic compounds having acidic protons, for example carboxylic acids or phenols. Basic organic compounds, for example based on amines, are also suitable.

The polyamide 10.10 (PA 10.10) usable in the present invention might be obtained by known melt polycondensation, processes of 1,10-decanediamine and decanedioic acid or from nylon salt solutions or nylon salt melts. The acid as well as the diamine may be produced from castor oil by processes known in the art. Preference is given to using regulated polyamide 10.10, preferably those in which the $NH_2$/COOH end group ratio of from 50 : 50 to 95 : 5 preferably of from 90 : 10 to 80 : 20 is present.

The solution of the polyamides for reprecipitation can be prepared in all known ways. What is advantageous is substantially complete dissolution of the polyamide in the alcoholic medium in the presence of the suspension of inorganic particles. The dissolution can be promoted by use of pressure and/or temperature. The procedure is appropriate to initially charge the polyamide in the alcoholic medium and to dissolve it over the time needed under the action of elevated temperature. The suspension of the inorganic particles can be added before, during or after the dissolution of the polyamide. Appropriately, the suspension of the inorganic particles is initially charged at the same time as the polyamide. The dissolution operation is favourably promoted by the use of appropriate stirrer units. The precipitation of the polyamide can likewise be supported by use of pressure and/or temperature. For instance, a lowering of the temperature and/or distillative removal (preferably under reduced pressure) of the solvent, i.e. of the alcoholic medium, lead to the precipitation of the polyamide. However, it is also possible to support the precipitation by addition of an antisolvent (precipitant).

It might be an advantage to post grind the particles based on polyamide 10.10 obtained by reprecipitation to adjust the mean particle size $d_{50}$. The post grinding can be done by methods known in the art.

The invention will be illustrated in detail below with reference to examples and comparative examples.

## Examples

**Example 1:** Preparation of a suspension

A 100 l stainless steel batch vessel is initially charged with 44 kg of ethanol and 1.00 kg of $H_3PO_4$ (85%). Subsequently, with running Ystral Conti-TDS 3 (stator slot: 4 mm ring and 1 mm ring, rotor/stator distance approx. 1 mm) under shear conditions, 21 kg of AEROXIDE® Alu C (BET 100 $m^2$/g) from Degussa are introduced into the batch vessel. Once approx. 18 kg of AEROXIDE® Alu C had been added, a further 0.13 kg of $H_3PO_4$ (85%) was added, in order again to achieve a low viscosity. Once the addition has ended, shearing is continued at 3000 rpm for another 30 min. At shear time 25 min, a further 1.2 kg of $H_3PO_4$ (85%) are added, so that a concentration of 11% $H_3PO_4$ (85%) based on the $Al_2O_3$ is achieved.

This presuspension is conducted in two passes through the Sugino Ultimaizer HJP-25050 high-energy mill at a pressure of 2500 bar and diamond dies of diameter 0.25 mm and thereby intensively ground further.

After the suspension, a particle size $d_{50}$ of 0.14 μm was determined by dynamic light scattering (Zetasizer 3000 Hsa from Malvern Instruments, UK). The volume-weighted median value of the peak analysis is reported.

**Example 2a:** Two-stage reprecipitation of amine terminated PA 10.10

50 kg of PA 10.10 obtained by polycondensation of 1,10-decanediamine and sebacic acid (decanedioic acid) having a relative solution viscosity of 1.65 (measured in 0.5% m-cresol solution at 25°C to DIN 53 727) and an end group content of 14 mmol/kg of COOH and 149 mmol/kg of $NH_2$ are brought to 155°C, in a 0.8 $m^3$ stirred tank together with 310 l of ethanol, denatured with 2-butanone and water content 1 % by weight, within 5 hours, and left at this temperature for 1 hour with stirring (paddle stirrer, d = 80 cm, speed = 90 rpm). Subsequently, the jacket temperature is reduced to 124°C and, while continuously distilling off the ethanol, the internal temperature is brought to 125°C with the same stirrer speed at a cooling rate of 25 K/h. From now on, the jacket temperature is kept 2 K - 3 K below the internal temperature at the same cooling rate. The internal temperature is brought to 125°C with the same cooling rate and then kept constant for 60 minutes. Thereafter, distillative removal is continued at a cooling rate of 40 K/h and the internal temperature is thus brought to 120°C. At this temperature, precipitation sets in, noticeable by the evolution of heat. The distillation rate is increased to such an extent that the internal temperature does not rise above 121.3°C. After 1 hour, the internal temperature falls, which indicates the end of precipitation. Further distillative removal and cooling via the jacket brings the temperature of the suspension to 45°C, and the suspension is then transferred to a paddle dryer.

The ethanol is distilled off at 70°C/400 mbar, and the residue is then dried at 20 mbar/86°C for 3 hours.

**Example 2b:** Two-stage reprecipitation of partially amine terminated PA 10.10

Example 2a is repeated with a PA1010 granulate having a relative solution viscosity of 1,68 and an end group content of 63 mmol/kg of COOH and 84 mmol/kg of $NH_2$. Product properties are shown in Table 1.

**Example 2c (not according to the invention):** Two-stage reprecipitation of partially amine terminated PA 10.10

Example 2a is repeated with a PA1010 granulate having a relative solution viscosity of 1,58 and an end group content of 43 mmol/kg of COOH and 104 mmol/kg of $NH_2$. The precipitation temperature is lowered by 2 K to 118°C. Product properties are shown in Table 1.

**Example 3:** One-stage reprecipitation of unregulated PA 1010 (relative solution viscosity of 1,69 and an end group content of 53 mmol/kg of COOH and 57 mmol/kg of $NH_2$) with addition of a suspension according to Example 1.

**Examples 3a - 3c**

The powders obtained in examples 2a,2b and 3 were post-ground in a jet mill type Hosokawa-Alpine 1250/6 AFG, the fine particle fraction passing the screen was collected, cf. table 1

50 kg of a PA 1010 specimen obtained by polycondensation of 1,10-decanediamine and sebacic acid and having a relative solution viscosity of 1.84 and an end group content of 62 mmol/kg of COOH and 55 mmol/kg of $NH_2$, together with 290 1 of ethanol denatured with 2-butanone and water content 1 % by weight and 17.4 kg of the suspension of example 1, are brought to 145°C in a 0.8 $m^3$ stirred tank within 5 hours and left at this temperature with stirring (paddle stirrer, d = 80 cm, speed = 90 rpm) for 1 hour. The jacket temperature is then reduced to 124°C and, while continuously distilling off the ethanol, the internal temperature is brought to 125°C with a cooling rate of 25 K/h at the same stirrer speed. From now on, the jacket temperature is kept 2 K - 3 K below the internal temperature at the same cooling rate until, at 120°C, precipitation, recognizable by the evolution of heat, sets in. The distillation rate is increased to such an extent that the internal temperature does not rise above 121.5 °C. After 20 minutes, the internal temperature declines, which indicates the end of the precipitation. Further distillative removal and cooling via the jacket brings the temperature of the suspension to 45°C, and the suspension is then transferred to a paddle dryer. The ethanol is distilled off at 70°C/ 500 mbar, and the residue is then dried at 20 mbar/86°C for 3 hours.

The particle parameters of the particles obtained in the examples 2 and 3 as well as the parameters of TEGOLON® 12-10, available from Evonik Goldschmidt GmbH are given in table 1. The parameters were obtained using the measurement techniques disclosed in the description.

**Table 1:** particle parameters

| Product | Type | BET $(m^2/g)$ | $d_{50}$ ($\mu m$) | AD g/l | Oil g/g |
|---|---|---|---|---|---|
| Example 2a | PA1010 | 45.3 | 41 | 262 | 3.20 |
| Example 2b | PA1010 | 17.0 | 44 | 272 | 2.20 |
| Example 2c | PA1010 | 3, 6 | 76 | 375 | 0.90 |
| Example 3 | PA1010 | 48.2 | 42 | 227 | 3.20 |
| Example 3a (post-ground powder from example 2a) | PA1010 | 16 | 11 | 231 | 2.35 |
| Example 3b (post-ground powder from example 2b) | PA1010 | 15 | 11 | 236 | 2.30 |
| Example 3c (post-ground powder from example 2c) | PA1010 | 3,1 | 14 | 310 | 1.00 |
| Example 3d (post-ground powder from example 3) | PA1010 | 21 | 10 | 243 | 2.50 |

(continued)

| Product | Type | BET (m$^2$/g) | d$_{50}$ ($\mu$m) | AD g/l | Oil g/g |
|---|---|---|---|---|---|
| TEGOLON® 12-10 | PA12 | 3.80 | 6.3 | 460 | 0.95 |

BET = surface area of the polyamide powder in m$^2$/g;
d$_{50}$ = particle size in $\mu$m;
AD = apparent density of the polyamide powder in g/l
Oil = oil absorption according to method described earlier.

**Example 4:** Comparison of inventive and non-inventive particles/formulations

As an illustration of the present invention, skin care compositions were prepared, one using the particles of example 2a, a second using the particles 3a, a third having a substantially similar formulation but with Tegolon® 12-10 particles and finally, a control formula with no PA particles at all.

To produce a face cream the ingredients as listed in table 2 were added in the following sequences: The water soluble components (phase A) and the oil soluble components (phase B) were heated separately to a temperature of about 85 °C under agitation. When the temperature of both phases has reached about 85 °C the components of each phase are thoroughly mixed and dissolved. Then phase A is slowly added to the oil phase B under agitation, followed by homogenization. The mixture is then cooled under gentle agitation to below about 40 °C and then sodium hydroxide solution, preservative and balance water are added. After complete mixing and additional cooling to room temperature the composition is ready for application.

The four formulations were evaluated for oily feeling, tackiness, absorption, smoothness and mattifying effects upon application to the skin. The subject composition which included the PA10.10 particles 2 a had a noticeable reduction in oiliness, tackiness, positive mattifying effect, and overall better skin feeling upon application as compared with the similar compositions having no PA10.10 microparticles or compositions comprising Tegolon 12-10.

The formulation with the PA10.10 according to example 3a, also showed a non-tacky, non-oily skin feel. Moreover, this test formula was described to be particularly smooth and its texture was rated to be of superior elegance.

Table 2: Cream compositions according to example 4

| Oil-in-Water Shine Control Face Cream | 4a | 4b | 4c | 4d |
|---|---|---|---|---|
| Glyceryl Stearate Citrate | 1.50% | 1.50% | 1.50% | 1.50% |
| Glyceryl Stearate | 2.00% | 2.00% | 2.00% | 2.00% |
| Ceteryl Alcohol | 3.00% | 3.00% | 3.00% | 3.00% |
| Caprylic/Capric Triglyceride | 7.30% | 7.30% | 7.30% | 7.30% |
| **C12-C15 Alkyl Benzoate | 10.50% | 10.50% | 10.50% | 10.50% |
| Carbomer | 0.20% | 0.20% | 0.20% | 0.20% |
| Octocrylene | 5.00% | 5.00% | 5.00% | 5.00% |
| Butyl Methoxydibenzoylmethane | 2.00% | 2.00% | 2.00% | 2.00% |
| Tocopheryl Acetate | 0.50% | 0.50% | 0.50% | 0.50% |
| Glycerin | 3.00% | 3.00% | 3.00% | 3.00% |
| Water | ad 100% | ad 100% | ad 100% | ad 100% |
| Example 2a (PA 10.10 particle) | | | 3.00% | |
| Example 3a (PA 10.10 particle) | | | | 3.00% |
| *Tegolon ® 12-10 | | 3.00% | | |

(continued)

| Oil-in-Water Shine Control Face Cream | 4a | 4b | 4c | 4d |
|---|---|---|---|---|
| Sodium Hydroxide (10 % in water) | 0.60% | 0.60% | 0.60% | 0.60% |
| Preservative, Perfume | q.s. | q.s. | q.s. | q.s. |
| Skin feel assessment | Oily, very tacky | Oily, tacky | Non-oily, non tacky, smooth & powdery feel | Non-oily, non tacky, extremely smooth and elegant texture |
| *Tegolon ® 12-10: Polyamide 12 powder of Evonik Goldschmidt GmbH<br>**TEGOSOFT® TN (Evonik Goldschmidt GmbH) | | | | |

**Example 5:** Comparison of inventive and non-inventive particles/formulations

In this example the examples of example 4 were repeated with the compositions as given in table 3.

Table 3: Cream composition according to example 5

| Oil-in-Water Cream Sebum Control | 5a | 5b | 5c | 5d |
|---|---|---|---|---|
| Glyceryl Stearate | 1.00% | 1.00% | 1.00% | 1.00% |
| Ceteryl Alcohol | 2.00% | 2.00% | 2.00% | 2.00% |
| Myristyl Myristate | 2.00% | 2.00% | 2.00% | 2.00% |
| Diethylhexyl Carbonate | 5.00% | 5.00% | 5.00% | 5.00% |
| Cyclomethicone | 5.00% | 5.00% | 5.00% | 5.00% |
| Isopropyl Palmitate | 2.00% | 2.00% | 2.00% | 2.00% |
| Saccharum officinarum (Sugar Cane) Extract | 0.50% | 0.50% | 0.50% | 0.50% |
| Cetearyl Glucoside | 1.50% | 1.50% | 1.50% | 1.50% |
| Propylene Glycol | 3.00% | 3.00% | 3.00% | 3.00% |
| Water | ad 100% | ad 100% | ad 100% | ad 100% |
| Example 3 (PA10.10) | | | 1.50% | |
| Example 3d (PA10.10) | | | | 1.50% |
| Tegolon ® 12-10 | | 1.50% | | |
| Carbomer | 0.20% | 0.20% | 0.20% | 0.20% |
| Isopropyl Palmitate | 0.80% | 0.80% | 0.80% | 0.80% |
| Ethanol | 2.00% | 2.00% | 2.00% | 2.00% |
| Sodium Hydroxide (10% in water) | q.s. | q.s. | q.s. | q.s. |
| Preservative, Perfume | q.s. | q.s. | q.s. | q.s. |
| | | | | |
| Skin feel assessment | Oily, very tacky | Oily, tacky | Non-oily, non tacky, smooth & powdery feel | Non-oily, quick absorption, extremely low tackiness, very smooth |

The four formulations were evaluated for oily feeling, tackiness and mattifying effects upon application to the skin. The subject compositions which included the PA10.10 particles had a noticeable reduction in oiliness, tackiness, positive mattifying effect, and overall better skin feeling upon application as compared with the similar compositions having no PA10.10 microparticles or compositions comprising Tegolon 12-10.

Especially the formulation with example 3d showed a particularly low oiliness, low tackiness and a quick absorption into the skin. Moreover, the skin feel of this test formula was rated to be particularly smooth.

**Examples 6 to 18**

The following examples 6 to 18 are the non-limiting examples of cosmetic formulations in which PA10.10 particles can be used. The formulations were produced in a similar way to the way described in example 4.

**Example 6:** Oil-in-Water Cream with Matt Finish

The composition of the cream is given in table 4.

Table 4: Composition of an Oil-in-Water Cream (% by weight)

| | |
|---|---|
| Polyglyceryl-3 Methylglucose Distearate | 3.00% |
| Glyceryl Stearate | 2.00% |
| Ceteryl Alcohol | 1.00% |
| ethylhexyl Stearate | 10.00% |
| Decyl Oleate | 9.00% |
| Example 2a (PA 10.10) | 1.50% |
| Glycerine | 3.00% |
| Water | Ad 100% |
| Preservative, Perfume | q.s. |

**Example 7:** Sheet Mask Impregnated Liquid

The composition of the sheet mask impregnated liquid is given in table 5 below.

Table 5: Composition of the sheet sask impregnated liquid (% by weight)

| | |
|---|---|
| TEGO ® Wipe DE | 5.70% |
| Cyclomethicone | 2.00% |
| Example 2b (PA 10.10) | 2.00% |
| Water | ad 100% |
| Glycerin | 3.00% |
| TEGO ® Carbomer 141 | 0.10%. |
| Sodium Hydroxide (10% in water) | q.s. |
| TEGO ® Wipe DE of Evonik Goldschmidt GmbH TEGO ® Carbomer 141 of Evonik Goldschmidt GmbH | |

**Example 8:** Two Way Powder Foundation

The composition of the two way powder foundation is given in table 6 below.

Table 6: Composition of the two way powder foundation (% by weight)

| | |
|---|---|
| Zinc Stearate | 3.00% |
| Sericite PHN Mica | 35.00% |

(continued)

| Talc | 24.00% |
|---|---|
| Mica | 10.00% |
| Example 3 (PA10.10) | 10.00% |
| Titanium Dioxide | 8.00% |
| Cetyl Ethylhexanoate | 2.00% |
| Squalane | 2.90% |
| Cetearyl Ethylhexanoate | 2.00%. |
| Mineral Oil (30 mPas) | 2.00% |
| PEG/PPG-4/12 Dimethicone | 1.00% |
| Preservative | 0.10% |
| Iron Oxides | q.s. |
| Perfume | q.s. |

**Example 9:** High Solid Cream-to-Powder Foundation Creamy Application with a Velvet finish

The composition of the high solid cream-to-powder foundation is given in table 7 below.

Table 7: Composition of the high solid cream-to-powder foundation (% by weight)

| Phenyl Trimethicone | 14.00% |
|---|---|
| Ethylhexyl Palmitate | 14.60% |
| Cetyl Ethylhexanoate | 5.00% |
| Carnauba Wax | 4.70% |
| Stearoxy Dimethicone | 4.00% |
| PVP/Eicosene Copolymer | 1.00% |
| Cetyl Stearyl Heptanoate | 2.85% |
| Covabead LH 85, Polymethylmethacrylates | 3.00% |
| *Silica | 0.25%. |
| Zinc Oxide | 7.00% |
| **Cyclopentasiloxane, Dimethicone Crosspolymer | 3.00% |
| Talc Covasil 4.05 | 9.50% |
| Acrylate Copolymer | 2.00% |
| Example 2a (PA10.10) | 2.00% |
| Alumnium Starch Octernylsuccinate | 9.50% |
| Iron Oxides | 3.10% |
| Titanium Dioxide (and) Dimethicone | 14.50% |
| *Aerosil 200 (Evonik Degussa GmbH) **Dow Corning 9040 silicone Elastomer Blend (Dow Corning) | |

**Example 10:** Volatile Silicone Cream Eye Shadow Stick

The composition of the volatile silicone cream eye shadow stick is given in table 8 below.

Table 8: Composition of the volatile silicone cream eye shadow stick (% by weight)

| Cyclomethicone | ad 100% |
|---|---|
| PPG-3 Myristyl Ether | 7.00% |
| *Polyglyceryl-4 Isostearate; Cetyl PEG/PPG-10/1 Dimethicone; Hexyl Laurate | 1.00% |
| Dimethicone (20 mPas) | 2.50% |
| Cera Alba | 4.50% |
| Carnauba Wax | 2.00% |
| *Lauryl Dimethicone/Polglyceryl-3 Crosspolymer, Triethylhexanoin | 2.00 |
| A-C Coploymer 400 (Ethylene/VA Copolymer | 2.50% |
| Ozokerite | 5.80% |
| C18-36 Acid Triglyceride | 2.00% |
| Liquipar Oil (Isobutylparaben (and) Isopropylparaben (and) Butylparaben) | 0.20% |
| Example 3 (PA10.10) | 2.00% |
| Titanium Dioxide | 5.00% |
| Chromium Oxide Green) | 10.00% |
| CI 77491 (and) Aluminum Powder (and) Silica | 5.00% |
| CI 77891 (and) CI 77288 (and) Mica | 10.00% |
| *ABIL ® WE 09 (Evonik Goldschmidt GmbH) **KSG-830 (Shin-Etsu) | |

**Example 11:** Compact Cream Rouge

The composition of the compact cream rouge is given in table 9 below.

Table 9: composition of the compact cream rouge (% by weight)

| Cylcopentasiloxane Trimethicone | 2.80% |
|---|---|
| Ceteryl Ethylhexanoate | 13.65% |
| Isopropyl Palmitate | 11.00% |
| Isopropyl Capylic/Capric Triglyceride | 11.25% |
| *Polyglyceryl-4 Isostearate; Cetyl PEG/PPG-10/1 Dimethicone; Hexyl Laurate | 2.10% |
| Cetyl Stearyl Heptanoate | 3.00% |
| Jojoba (Buxus Chinensis) Oil | 3.75% |
| Petrolatum | 2.75% |
| Carnauba Wax 2442 L (Carnauba Wax) | 1.65%. |
| Candelilla Wax 2039 Y (Candelilla Wax) | 2.20% |
| C8-C36 Acid Triglyceride | 2.10% |
| Covabead LH 85 (Polymethylmethacrylates) | 13.65% |
| Talc Covasil 4.05 (Talc; Dimethicone; Trimethylsiloxysilicate | 9.10% |
| Titanium Dioxide (and) CI 77891 | 2.95% |
| Iron Oxides | 0.50% |
| Soft-Tex Yellow C 33-7715 | 0.60% |

(continued)

| | |
|---|---|
| Soft Tex Brown C33-7715 | 1.05% |
| D&C Red No. 30 Alumnium Lake) | 0.45% |
| CI 77941 (and) Silica | 13.60% |
| Example 3 (PA 10.10) | 1.85% |
| *ABIL ® WE 09 (Evonik Goldschmidt GmbH) | |

**Example 12:** Sun Care Cream

The composition of the sun care cream is given in table 10 below.

Table 10: Composition of the sun care cream (% by weight)

| | |
|---|---|
| *Ceteareth-15; Glyceryl Stearate | 2.50% |
| Stearyl Alcohol | 2.00% |
| Caprylic/Capril Triglyceride | 4.50% |
| Octocrylene | 1.00% |
| Bis-Ethylhexyloxyphenol Methoxyphenyl Triazine | 4.00% |
| Homomenthyl Salicylate | 1.00% |
| **Tego® Sun T 805 | 6.50% |
| Xanthum Gum | 0.40% |
| Glycerin | 3.00%. |
| Water | Ad 100% |
| Example 3 (PA10.10) | 3.00% |
| Preservative | q.s. |
| Perfume | q.s. |
| **Tego® care 215 (Evonik Goldschmidt GmbH) *Tego® Sun T 805 (Evonik Goldschmidt GmbH) | |

**Example 13:** Sun Care Lotion

The composition of the sun care lotion is given in table 11 below.

Table 11: Sun care lotion composition (% by weight)

| | |
|---|---|
| Glyceryl Sterate Citrate | 3.00% |
| Cetearyl alcohol | 1.00% |
| Cetyl Dimethicone | 0.20% |
| **$C_{12}$-$C_{15}$ Alkyl Benzoate | 4.80% |
| Triisostearin | 1.00% |
| Diethylhexyl Carbonate | 6.00% |
| *Tego® Sun T 805 | 3.00% |
| Tocopheryl Acetate | 0.50% |
| Ethylhexyl Methoxycinnamate | 5.00%. |
| Butyl Methoxydibenzoylmethane | 2.50% |

(continued)

| Carbober | 0.20% |
|---|---|
| Xanthum Gum | 0.40% |
| Sodium Carboxymethyl Betaglucan | 0.10% |
| Glycerin | 2.00% |
| Water | ad 100% |
| Example 3 (PA10.10) | 1.50% |
| Sodium Hydroxide (10% in water) | q.s. |
| Perfume | q.s. |
| *Tego® Sun T 805<br>**TEGOSOFT® TN (Evonik Goldschmidt GmbH) | |

**Example 14:** Anti- perspirant/Deo Roll-on

The composition of the Anti-perspirant/Deo Roll-on is given in table 12 below.

Table 12: Anti-perspirant/Deo Roll-on composition (% by weight)

| Stearath-2 | 2.20% |
|---|---|
| Stearath-20 | 1.00% |
| Cetearyl Ethylhexanoate | 2.00% |
| PPG-11 Stearyl Ether | 2.00% |
| Dimethicone | 0.50% |
| Polyglyceryl 3-Caprylate | 0.50% |
| Water | ad 100% |
| Glycerin | 3.00% |
| Example 2a (PA10.10) | 0.30%. |
| Perfume | q.s. |
| Citric Acid (50% in water) | q.s. |
| Preservative | q.s. |

**Example 15:** Hair Repair Leave-in Conditioner

The composition of the hair repair leave-in Conditioner is given in table 13 below.

Table 13: Hair repair leave-in conditioner composition (% by weight)

| PEG-40 Hydrogenated Castor Oil | 2.00% |
|---|---|
| Ceramide 6 II | 0.05% |
| Perfume | 0.20% |
| Water | ad 100% |
| Example 2a (PA10.10) | 2.00% |
| *Sodium Lactate; Sodium PCA; Glycine; Fructose; Urea; Niacinamide; Inositol; Sodium benzoate; Lactic Acid | 2.00% |
| Cocamidopropyl Betaine | 2.00% |

(continued)

| Citric Acid (10% in water) | q.s. |
|---|---|
| *Lactil® (Evonik Degussa GmbH) | |

**Example 16:** 2 in 1 Shampoo

The composition of the 2-in-1 shampoo is given in table 14 below.

Table 14: 2-in-1 shampoo composition (% by weight)

| Sodium Laureth Sulfate | 32,00% |
|---|---|
| Palmitamidopropyltrimonium Chloride | 1,50% |
| *PEG-200 Hydrogenated Glyceryl Palmate; PEG-7 Glyceryl Cocoate) | 2,00% |
| Example 2a (PA10.10) | 0,50% |
| Perfume | 0,25% |
| Water | ad 100% |
| Creatine | 1,00% |
| Hydroxypropyl Guar Hydroxypropyltrimonium Chloride | 0,20 |
| Cocamidopropyl Betaine | 8,00% |
| NaCl | 0,50% |
| Preservative | q.s. |
| *Rewoderm® LI S 80 (Evonik Goldschmidt GmbH) | |

**Example 17:** Rinse-off conditioner

The composition of the conditioner is given in table 15 below.

Table 15: Conditioner composition (% by weight)

| Water | ad 100% |
|---|---|
| *Distearyl Dimonium Chloride, Cetearyl Alcohol | 2,00% |
| Behentrimonium Chloride | 2,00% |
| Quaternium-80 | 1,00% |
| Example 3 (PA10.10) | 0,80% |
| Cetearyl Alcohol | 5,00% |
| Preservative, Perfume | q.s. |
| *VARISOFT® EQ 65 (Evonik Goldschmidt GmbH) | |

**Example 18:** Conditioning Rinse

The composition of the conditioning rinse is given in table 16 below.

Table 16: Conditioning rinse composition (% by weight)

| Water | ad 100% |
|---|---|
| *Distearoylethyl Dimonium Chloride; Cetearyl Alcohol | 2,0% |
| Behentrimonium Chloride | 2,0% |
| Example 2a (PA10.10) | 2,0% |

(continued)

| Silicone Quaternium-22 | 0,8% |
|---|---|
| Cetearyl Alcohol | 5,0% |
| Preservative, Perfume | q.s. |
| *VARISOFT® EQ 65 (Evonik Goldschmidt GmbH) | |

**Claims**

1. Particles based on polyamide 10.10, **characterized in that** the particles have a mean particle size $d_{50}$ as determined as described in the description of from 1 to 50 $\mu$m,
an apparent density according to DIN 53644 of from 180 to 300g/l and
a $NH_2$/COOH end group ratio as determined by alkalimetric titration with KOH in hot benzylic alcohol at 180°C and by acidimetric titration with $HClO_4$ at ambient temperature in m-Cresol of from 50 : 50 to 95 : 5.

2. Particles as claimed in claim 1, **characterized in that** the pH of the polyamide 10.10 is of from 2 to 7.

3. Particles as claimed in claim 1 or 2, **characterized in that** they comprise a buffer system or at least the remains of a buffer system.

4. Particles as claimed in claim 3, **characterized in that** the buffer system comprises an organic acid or a mineral acid and a corresponding salt thereof.

5. Particles as claimed in any of the claims 1 to 4, **characterized in that** they show a BET surface as determined as described in the description of from 1 to 20 $m^2$/g.

6. Particles as claimed in any of the claims 1 to 4, **characterized in that** they show a BET surface of from more than 20 to 50 $m^2$/g.

7. Particles as claimed in any of the claims 1 to 6, **characterized in** a mean number average molecular weight of the polyamide 10.10 of from 5000 to 50000 g/mol.

8. Particles as claimed in any of claims 1 to 7, **characterized in** a content of inorganic particles of from 0.1 to 80 % by weight, based on the total weight of the particles.

9. Formulation, especially cosmetic, dermatologic or pharmaceutical formulation comprising 0,1 to 20 % by weight of particles according to one of the claims 1 to 8.

10. Formulation according to claim 9, **characterized in that** the composition is selected from powder composition, foundation, nail polish, aerosol, lipstick, eye shadow, masking stick, rouge, skin cream, face cream, hair care formulation, sun care formulation, cleansing formulation and antiperspirant/deo formulation.

11. Use of particles according to any of claims 1 to 8 for producing a cosmetic, dermatologic or pharmaceutical formulation, preferably selected from powder composition, foundation, nail polish, aerosol, lipstick, eye shadow, masking stick, rouge, skin cream and face cream hair care formulation, sun care formulation, cleansing formulation and antiperspirant/deo formulation.

12. Process for preparing particles according to any of claim 1 to 8 by dissolving polyamide 10.10, having a relative solution viscosity $\eta_{rel}$ in the range from 1.4 to 2.0, measured in 0.5% m-cresol solution at 25°C, in an alcoholic medium, lowering the temperature in a first stage until nucleation takes place without precipitation, lowering the temperature further in a second stage until supersaturation results, precipitating said polyamide powder and drying the resulting suspension, wherein said polyamide 10.10 is dissolved at from 130 to 165 °C and precipitation is carried out isothermally at a precipitation temperature of from 100 to 130 °C preceded by a nucleation stage at from 2 to 20 °C above said precipitation temperature, wherein said temperature during precipitation is held constant for from 10 minutes to 2 hours, and wherein said temperature during nucleation is held constant for from 30 to 90 minutes.

**13.** Process according to claim 12, **characterized in that** in the dissolving step 0.1 to 80 % by weight, of inorganic particles are added to the alcoholic medium, based on the total weight of the sum of polyamide 10.10 and inorganic particles used.

**14.** Process as claimed in claim 12 or 13, **characterized in** the use of inorganic particles selected from the group of $Al_2O_3$, $TiO_2$, $ZrO_2$, $SiO_2$, ZnO, $Bi_2O_3$, $CeO_2$, ITO, ATO, IZO, boron nitride, boron carbide, mixed oxides and spinels, having a mean particle size $d_{50}$ of from 0.001 to 0.8 $\mu$m.

**15.** Process according to at least one of the claims 12 to 14, **characterized in that** the inorganic particles are added to the medium by adding a suspension comprising alcohol and the inorganic particles, wherein a suspension is used which has a content of inorganic particles in the range from 10 to 60% by weight based on the total weight of the suspension.

**Patentansprüche**

**1.** Partikel auf 10.10-Polyamidbasis, **dadurch gekennzeichnet, dass** die Partikel eine durchschnittliche Partikelgröße $d_{50}$, die wie in der Beschreibung beschrieben bestimmt wird, von 1 bis 50 $\mu$m,
ein Schüttgewicht gemäß DIN 53644 von 180 bis 300 g/L und
ein $NH_2$/COOH-Endgruppenverhältnis, das durch alkalimetrische Titration mit KOH in heißem Benzylalkohol bei 180 °C und durch acidimetrische Titration mit $HClO_4$ bei Umgebungstemperatur in m-Kresol von 50:50 bis 95:5 bestimmt wird, aufweisen.

**2.** Partikel nach Anspruch 1, **dadurch gekennzeichnet, dass** der pH-Wert des 10.10-Polyamids von 2 bis 7 beträgt.

**3.** Partikel nach Anspruch 1 oder 2, **dadurch gekennzeichnet, dass** diese ein Puffersystem oder zumindest die Reste eines Puffersystems umfassen.

**4.** Partikel nach Anspruch 3, **dadurch gekennzeichnet, dass** das Puffersystem eine organische Säure oder eine Mineralsäure und ein entsprechendes Salz davon umfasst.

**5.** Partikel nach einem der Ansprüche 1 bis 4, **dadurch gekennzeichnet, dass** diese eine BET-Oberfläche, die wie in der Beschreibung beschrieben bestimmt wird, von 1 bis 20 $m^2$/g aufweisen.

**6.** Partikel nach einem der Ansprüche 1 bis 4, **dadurch gekennzeichnet, dass** diese eine BET-Oberfläche von mehr als 20 bis 50 $m^2$/g aufweisen.

**7.** Partikel nach einem der Ansprüche 1 bis 6, **gekennzeichnet durch** ein zahlengemitteltes durchschnittliches Molekulargewicht des 10.10-Polyamids von 5000 bis 50.000 g/Mol.

**8.** Partikel nach einem der Ansprüche 1 bis 7, **gekennzeichnet durch** einen Gehalt anorganischer Partikel von 0,1 bis 80 Gew.-%, basierend auf dem Gesamtgewicht der Partikel.

**9.** Formulierung, insbesondere kosmetische, dermatologische oder pharmazeutische Formulierung, umfassend 0,1 bis 20 Gew.-% der Partikel nach einem der Ansprüche 1 bis 8.

**10.** Formulierung nach Anspruch 9, **dadurch gekennzeichnet, dass** die Zusammensetzung ausgewählt ist aus einer Pulverzusammensetzung, Grundierung, Nagellack, Aerosol, Lippenstift, Lidschatten, Abdeckstift, Rouge, Hautcreme, Gesichtscreme, Haarpflegeformulierung, Sonnenschutzformulierung, Reinigungsformulierung und Antitranspirant-/Deodorant-Formulierung.

**11.** Verwendung der Partikel nach einem der Ansprüche 1 bis 8 zum Herstellen einer kosmetischen, dermatologischen oder pharmazeutischen Formulierung, die vorzugsweise ausgewählt ist aus einer Pulverzusammensetzung, Grundierung, Nagellack, Aerosol, Lippenstift, Lidschatten, Abdeckstift, Rouge, Hautcreme und Gesichtscreme, Haarpflegeformulierung, Sonnenschutzformulierung, Reinigungsformulierung und Antitranspirant-/Deodorant-Formulierung.

**12.** Verfahren zum Herstellen von Partikel nach einem der Ansprüche 1 bis 8 durch Lösen von 10.10-Polyamid, das

eine relative Lösungsviskosität $\eta_{rel}$ im Bereich von 1,4 bis 2,0 aufweist, die in 0,5 % m-Kresollösung bei 25 °C in einem alkoholischen Medium gemessen wird, Senken der Temperatur in einer ersten Stufe, bis die Keimbildung ohne Ausfällung stattfindet, weiteres Senken der Temperatur in einer zweiten Stufe, bis sich eine Übersättigung ergibt, Ausfällen des Polyamidpulvers und Trocknen der resultierenden Suspension, wobei das 10.10-Polyamid bei 130 bis 165 °C gelöst wird und die Ausfällung isotherm bei einer Ausfällungstemperatur von 100 bis 130 °C durchgeführt wird, wobei dieser eine Keimbildungsstufe bei 2 bis 20 °C über dieser Ausfällungstemperatur vorausgeht, wobei die Temperatur während der Ausfällung 10 Minuten bis 2 Stunden lang konstant gehalten wird und wobei die Temperatur während der Keimbildung 30 bis 90 Minuten lang konstant gehalten wird.

13. Verfahren nach Anspruch 12, **dadurch gekennzeichnet, dass** in dem Löseschritt 0,1 bis 80 Gew.-% der anorganischen Partikel, bezogen auf das Gesamtgewicht der Summe von 10.10-Polyamid und den verwendeten anorganischen Partikel, dem alkoholischen Medium zugegeben werden.

14. Verfahren nach Anspruch 12 oder 13, **gekennzeichnet durch** die Verwendung von anorganischen Partikel, die ausgewählt sind aus der Gruppe von $Al_2O_3$, $TiO_2$, $ZrO_2$, $SiO_2$, ZnO, $Bi_2O_3$, $CeO_2$, ITO, ATO, IZO, Bornitrid, Borcarbid, gemischten Oxiden und Spinellen mit einer durchschnittlichen Partikelgröße $d_{50}$ von 0,001 bis 0,8 $\mu$m.

15. Verfahren nach mindestens einem der Ansprüche 12 bis 14, **dadurch gekennzeichnet, dass** die anorganischen Partikel dem Medium durch Zugeben einer Suspension zugegeben werden, die Alkohol und die anorganischen Partikel umfasst, wobei eine Suspension verwendet wird, die einen Gehalt an anorganischen Partikel im Bereich von 10 bis 60 Gew.-% basierend auf dem Gesamtgewicht der Suspension aufweist.

## Revendications

1. Particules à base de polyamide 10.10, **caractérisées en ce que** les particules ont une taille moyenne de particules $d_{50}$ telle que déterminée comme décrit dans la description de 1 à 50 $\mu$m,
une densité apparente selon DIN 53644 de 180 à 300 g/l et
un rapport de groupes terminaux $NH_2$/COOH tel que déterminé par titrage alcalimétrique avec du KOH dans de l'alcool benzylique chaud à 180° et par titrage acidimétrique avec de l'$HClO_4$ à la température ambiante dans du m-Crésol de 50:50 à 95:5.

2. Particules selon la revendication 1, **caractérisées en ce que** le pH du polyamide 10.10 est de 2 à 7.

3. Particules selon la revendication 1 ou 2, **caractérisées en ce qu'**elles comprennent un système tampon ou au moins les résidus d'un système tampon.

4. Particules selon la revendication 3, **caractérisées en ce que** le système tampon comprend un acide organique ou un acide minéral et un sel correspondant de celui-ci.

5. Particules selon l'une quelconque des revendications 1 à 4, **caractérisées en ce qu'**elles présentent une surface BET telle que déterminée comme décrit dans la description de 1 à 20 m$^2$/g.

6. Particules selon l'une quelconque des revendications 1 à 4, **caractérisées en ce qu'**elles présentent une surface BET de plus de 20 à 50 m$^2$/g.

7. Particules selon l'une quelconque des revendications 1 à 6, **caractérisées par** un poids moléculaire moyen en nombre du polyamide 10.10 de 5000 à 50 000 g/mol.

8. Particules selon l'une quelconque des revendications 1 à 7, **caractérisées par** une teneur en particules minérales de 0,1 à 80 % en poids, sur la base du poids total des particules.

9. Formulation, notamment formulation cosmétique, dermatologique ou pharmaceutique, comprenant de 0,1 à 20 % en poids de particules selon l'une quelconque des revendications 1 à 8.

10. Formulation selon la revendication 9, **caractérisée en ce que** la composition est choisie parmi une composition en poudre, un fond de teint, un vernis à ongle, un aérosol, un rouge à lèvre, un fard à paupières, un masque en bâton, un rouge à joues, une crème pour la peau, une crème pour le visage, une formulation de soin capillaire, une formulation de soin solaire, une formulation de nettoyage et une formulation antitranspirante/déo.

11. Utilisation de particules selon l'une quelconque des revendications 1 à 8, pour la production d'une formulation cosmétique, dermatologique ou pharmaceutique, de préférence choisie parmi une composition en poudre, un fond de teint, un vernis à ongle, un aérosol, un rouge à lèvre, un fard à paupières, un masque en bâton, un rouge à joues, une crème pour la peau et une crème pour le visage, une formulation de soin capillaire, une formulation de soin solaire, une formulation de nettoyage et une formulation antitranspirante/déo.

12. Procédé de préparation de particules selon l'une quelconque des revendications 1 à 8, par la dissolution du polyamide 10.10, ayant une viscosité relative en solution $\eta_{rel}$ dans la gamme de 1,4 à 2,0, mesurée dans une solution de m-crésol à 0,5 % à 25°C, dans un milieu alcoolique, la réduction de la température dans une première étape jusqu'à ce qu'une nucléation se produise sans précipitation, la réduction de la température de nouveau dans une deuxième étape jusqu'à ce qu'une sursaturation se produise, la précipitation de ladite poudre de polyamide et le séchage de la suspension résultante, ledit polyamide 10.10 étant dissous à une température de 130 à 165 °C et la précipitation étant effectuée de façon isothermique à une température de précipitation de 100 à 130 °C précédée d'une étape de nucléation à une température de 2 à 20 °C au-dessus de ladite température de précipitation, ladite température pendant la précipitation étant maintenue constante pendant 10 minutes à 2 heures, et ladite température pendant la nucléation étant maintenue constante pendant 30 à 90 minutes.

13. Procédé selon la revendication 12, **caractérisé en ce que** pendant l'étape de dissolution, on ajoute au milieu alcoolique de 0,1 à 80 % en poids de particules minérales, sur la base du poids total de la somme du polyamide 10.10 et des particules minérales utilisés.

14. Procédé selon la revendication 12 ou 13, **caractérisé par** l'utilisation de particules minérales choisies dans le groupe de $Al_2O_3$, $TiO_2$, $ZrO_2$, $SiO_2$, ZnO, $Bi_2O_3$, $CeO_2$, ITO, ATO, IZO, nitrure de bore, carbure de bore, oxydes mixtes et spinelles, ayant une taille moyenne de particules $d_{50}$ de 0,001 à 0,8 $\mu$m.

15. Procédé selon au moins l'une des revendications 12 à 14, **caractérisé en ce que** les particules minérales sont ajoutées au milieu par l'ajout d'une suspension comprenant de l'alcool et des particules minérales, une suspension étant utilisée qui a une teneur en particules minérales dans la gamme de 10 à 60 % en poids sur la base du poids total de la suspension.

EP 2 374 835 B1

**Patent documents cited in the description**

- US 20030114636 A
- EP 0863174 A
- US 5932687 A
- EP 1726610 A
- EP 1834979 A
- US 20080249237 A
- DE 10161038 A1
- DE 102008001788
- US 2003053970 A
- US 2008024937 A
- DE 4421454
- DE 10360766
- DE 102005032427

**Non-patent literature cited in the description**

- **P. FINKEL.** *SÖFW-Journal,* 1996, vol. 122, 543